(19) Europäisches Patentamt
European Patent Office
Office européen des brevets

(11) Publication number: **0 096 569**
**A2**

# (12) EUROPEAN PATENT APPLICATION

(21) Application number: 83303244.4

(22) Date of filing: 06.06.83

(51) Int. Cl.³: C 07 D 249/08
A 61 K 31/41, A 01 N 43/64

(30) Priority: 09.06.82 GB 8216746
30.07.82 GB 8222026

(43) Date of publication of application:
21.12.83 Bulletin 83/51

(84) Designated Contracting States:
AT BE CH DE FR GB IT LI LU NL SE

(71) Applicant: Pfizer Limited
Ramsgate Road
Sandwich Kent CT13 9NJ(GB)

(84) Designated Contracting States:
GB

(71) Applicant: Pfizer Corporation
Calle 15 1/2 Avenida Santa Isabel
Colon(PA)

(84) Designated Contracting States:
BE CH DE FR IT LI LU NL SE AT

(72) Inventor: Narayanaswami, Subramaniyan, Dr.
31 Bruce Close
Deal Kent(GB)

(72) Inventor: Richardson, Kenneth, Dr.
48 St. Stephens Hill
Canterbury Kent(GB)

(74) Representative: Wood, David John et al,
Pfizer Limited Ramsgate Road
Sandwich Kent CT13 9NJ(GB)

## (54) Triazole antifungal agents.

(57) A triazole antifungal agent of the formula:-

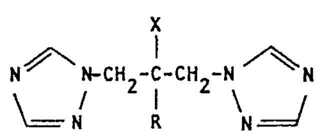

---(I)

or a pharmaceutically or agriculturally acceptable salt thereof, wherein R is naphthyl, biphenylyl, or phenyl optionally substituted by 1 to 3 substituents each independently selected from F, Cl, Br, I, $CF_3$, $C_1$-$C_4$ alkyl and $C_1$-$C_4$ alkoxy; or R is a 5-chloropyrid-2-yl group;

and X is F, Cl or Br; processes for their production; and pharmaceutical and plant or seed antifungal compositions containing them.

The compounds are useful as antifungal agents for human and agricultural use.

EP 0 096 569 A2

This invention relates to novel bis-triazole derivatives which have antifungal activity and are useful in the treatment of fungal infections in animals, including humans, and as agricultural fungicides.

According to the invention, there are provided compounds of the formula:-

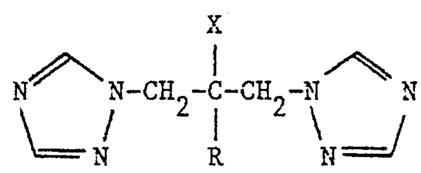

--- (I)

whre R is naphthyl, biphenylyl, or phenyl optionally substituted by 1 to 3 substituents each independently selected from F, Cl, Br, I, $CF_3$, $C_1$-$C_4$ alkyl and $C_1$-$C_4$ alkoxy; or R is a 5-chloro-pyrid-2-yl group; and X is F, Cl or Br; and their pharmaceutically and agriculturally acceptable salts.

Preferably, R is phenyl substitued by 1 to 3 substituents, most preferably 1 or 2 substituents, each independently selected from F, Cl, Br, I and $CF_3$.

In particular, R is phenyl substituted by 4-chloro, 4-bromo, 4-fluoro, 4-iodo, 4-trifluoromethyl, 2-chloro, 2,4-dichloro, 2,4-difluoro, 2-chloro-4-fluoro, 2,5-difluoro, 2,4,6-trifluoro or 4-bromo-2,5-difluoro.

Most preferably, R is 2,4-dichlorophenyl, 2,4-difluorophenyl, 4-bromo-2,5-difluorophenyl, 4-iodophenyl, 4-chlorophenyl or 5-chloropyrid-2-yl.

$C_3$ and $C_4$ alkyl and alkoxy groups can be straight or branched chain.

The preferred biphenylyl group is 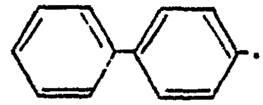.

The most preferred individual compound is 1,3-bis(1H-1,2,4-triazol-1-yl)-2-bromo-2-(2,4-dichlorophenyl)propane.

The compounds of the formula (I) can be prepared by the halogenation of the corresponding hydroxy compounds of the formula:-

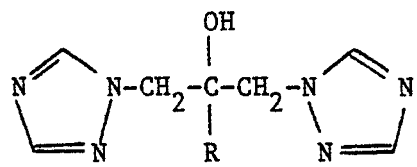

--- (II).

The halogenation is carried out according to conventional procedures, e.g. using $SOCl_2$, $SOBr_2$ or diethylaminosulphur trifluoride ($Et_2NSF_3$) as appropriate.

In a typical procedure utilising thionyl chloride or bromide, the propanol (II) in a suitable solvent, e.g. dry acetonitrile, is reacted with thionyl chloride or bromide at a temperature of from 0°C to reflux temperature, optionally in the presence of a base, e.g. imidazole. The product (I) can then be isolated and purified in a conventional manner.

The reaction using diethylaminosulphur trifluoride is typically carried out at a temperature of from about 0°C to room temperature, preferably in methylene chloride as the solvent. Again the product can be isolated and purified conventionally.

Many of the starting materials of the formula (II) are known compounds (see published British Patent Applications nos. 2,099,818A and 2,078,719A). The starting materials of the formula (II) in which R is 5-chloropyrid-2-yl are described and claimed in our copending U.K. application no. 8217114.

Typical methods to the starting materials are as follows:-

(a)

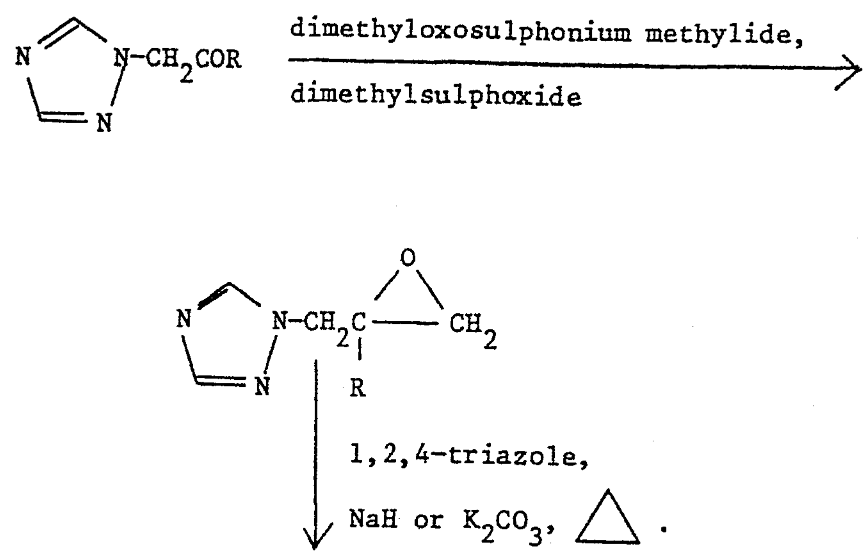


Compound (II)

The methylide can be prepared from trimethylsulphoxonium iodide and either sodium hydride or cetrimide/sodium hydroxide,

and (b)

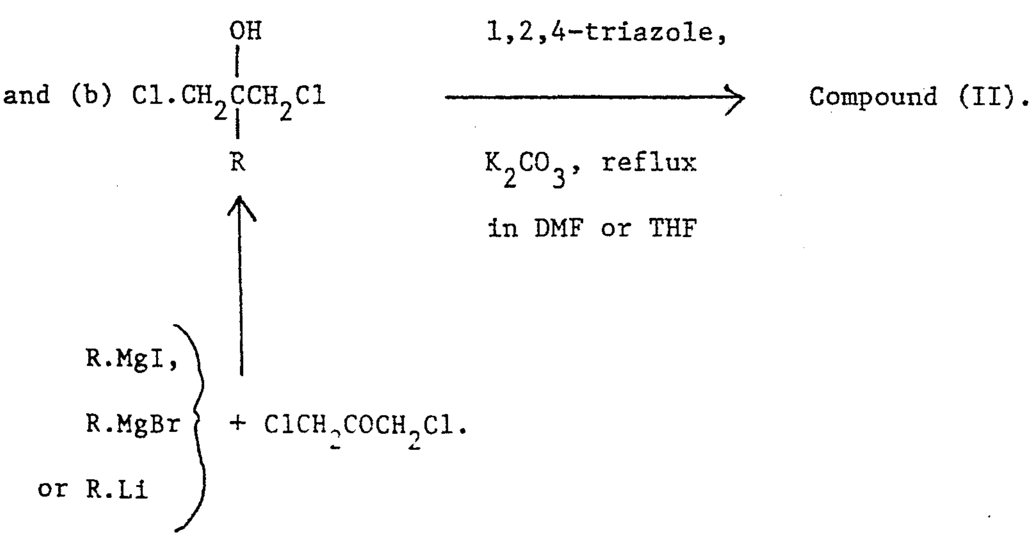

The invention also provides a pharmaceutical composition comprising a compound of the formula (I) or a pharmaceutically acceptable salt thereof, together with a pharmaceutically acceptable diluent or carrier. The composition is preferably for human use and in tablet, capsule, injectable or ointment form.

The invention yet further provides a plant or seed antifungal composition comprising a compound of the formula (I), or an agriculturally acceptable salt thereof, together with an agriculturally acceptable diluent or carrier.

In addition the invention provides a method of treating an animal, (including a human being), plant or seed having a fungal infection, which comprises treating said animal, plant or seed with an effective amount of a compound of the formula (I) or with, respectively, a pharmaceutically or agriculturally acceptable salt thereof.

The invention further provides a compound of the formula (I), or a pharmaceutically acceptable salt thereof, for use in treating a fungal infection in a human being.

The preferred pharmaceutically acceptable salts are the acid addition salts. Pharmaceutically acceptable acid addition salts of the compounds of the formula (I) are those formed from strong acids which form non-toxic acid addition salts containing pharmaceutically-acceptable anions, such as the hydrochloride, hydrobromide and sulphate.

The salts may be obtained by conventional procedures, e.g. by mixing solutions containing equimolar amounts of the free base and desired acid, and the required salt is collected by filtration, if insoluble, or by evaporation of the solvent.

The compounds of the formula (I) and their pharmaceutically acceptable salts are very active arti-fungal agents, useful in combating fungal infections in animals, including humans. For example they are useful in treating topical fungal infections in man caused by, among other organisms, species of Candida, Trichophyton, Microsporum, or Epidermophyton, or in mucosal infections caused by Candida albicans (e.g. thrush and vaginal candidiasis). They may also be used in the treatment of systemic fungal infections caused by, for example, Candida albicans, Cryptococcus neoformans, Aspergillus fumigatus, Coccidioides, Paracoccidioides, Histoplasma or Blastomyces.

The in vitro evaluation of the antifungal activity of the compounds (I) can be performed by determining the minimum inhibitory concentration (m.i.c.) which is the concentration of the test compound in a suitable medium at which growth of the particular microorganism fails to occur. In practice, a series of agar plates, each having the test compound incorporated at a particular concentration are inoculated with a standard culture of, for example, Candida albicans and each plate is then incubated for 48 hours at 37°C. The plates are then examined for the presence or absence or growth of the fungus and the appropriate m.i.c. value is noted. Other microorganisms used in such tests can include Cryptococcus neoformans, Aspergillus fumigatus, Trichophyton spp, Microsporum spp, Epidermophyton floccosum, Coccidioides immitis, and Torulopsis glabrata.

The in vivo evaluation of the compounds can be carried out at a series of dose levels by intraperitoneal or intravenous injection or by oral administration, to mice which are inoculated

with a strain of Candida albicans. Untreated mice die within 48 hours and the dose level at which the compound provides 50% protection against the lethal effect of the infection ($PD_{50}$) is noted.

For human use, the antifungal compounds of the formula (I) (or salts thereof) can be administered alone, but will generally be administered in admixture with a pharmaceutical carrier selected with regard to the intended route of administration and standard pharmaceutical practice. For example, they may be administered orally in the form of a tablet containing such excipients as starch or lactose, or in a capsule or ovule either alone or in admixture with excipients, or in the form of an elixir or suspension containing a flavouring or colouring agent. They may be injected parenterally, for example, intravenously, intramuscularly or subcutaneously. For parenteral administration, they are best used in the form of a sterile aqueous solution which may contain other substances, for example, enough salts or glucose to make the solution isotonic.

For oral and parenteral administration to human patients, it is expected that the daily dosage level of the antifungal compounds of the formula (I) will be from 0.1 to 5 mg/kg (in divided doses) when administered by either the oral or parenteral route. Thus tablets or capsules of the compounds can be expected to contain from 5 mg to 0.5 g of active compound for administration singly or two or more at a time as appropriate. The physician in any event will determine the actual dosage which will be most suitable for an individual patient and it will vary with the age, weight and response of the particular patient. The

above dosages are exemplary of the average case. There can, of course, by individual instances where higher or lower dosage ranges are merited, and such are within the scope of this invention.

Alternatively, the antifungal compounds of formula (I) can be administered in the form of a suppository or pessary, or they may be applied topically in the form of a lotion, solution, cream, ointment or dusting powder. For example, they can be incorporated into a cream consisting of an aqueous emulsion of polyethylene glycols or liquid paraffin; or they can be incorporated, at a concentration between 1 and 10%, into an ointment consisting of a white wax or white soft paraffin base together with such stabilizers and preservatives as may be required.

The compounds of the formula (I) and their salts also have activity against a variety of plant pathogenic fungi, including for example various rusts, mildews and moulds, and the compounds are thus useful for treating plants and seeds to eradicate or prevent such diseases.

The *in vitro* evaluation of the activity of the compounds against plant fungi can be determined by measuring their minimum inhibitory concentrations in the same way as previously described except that the plates are incubated at 30°C for 48 hours or longer before being examined for the presence or absence of growth.

Micro-organisms used in such tests include *Cochliobolus carbonum*, *Pyricularia oryzae*, *Glomerella cingulata*, *Penicillium digitatum*, *Botrytis cinerea* and *Rhizoctonia solani*.

For agricultural and horticultural purposes the compounds and their agriculturally acceptable salts are preferably used in the form of a composition formulated as appropriate to the particular use and purpose desired. Thus the compounds may be applied in the form of dusting powders, or granules, seed dressings, aqueous solutions, dispersions or emulsions, dips, sprays, aerosols or smokes. Compositions may also be supplied in the form of dispersible powders, granules or grains, or concentrates for dilution prior to use. Such compositions may contain such conventional carriers, diluents or adjuvants as are known and acceptable in agriculture and horticulture and they are manufactured in accordance with conventional procedures. The compositions may also incorporate other active ingredients, for example, compounds having herbicidal or insecticidal activity or a further fungicide. The compounds and compositions can be applied in a number of ways, for example they can be applied directly to the plant foliage, stems, branches, seeds or roots or to the soil or other growing medium, and they may be used not only to eradicate disease, but also prophylactically to protect the plants or seeds from attack.

The following Examples illustrate the invention. All temperatures are in °C.

EXAMPLE 1

1,3-Bis-(1H-1,2,4-triazol-1-yl)-2-chloro-2-(2,4-dichlorophenyl) propane

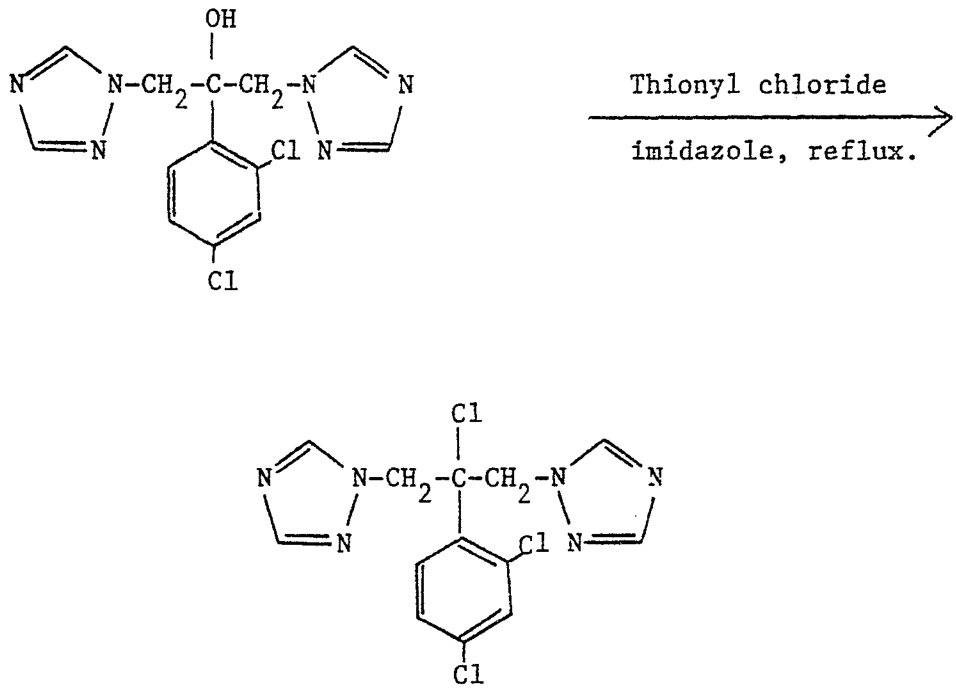


A solution of 1.94 g of imidazole in 12 ml of dry acetonitrile was treated with 1 ml of thionyl chloride and then with 1.63 g of 1,3-bis-(1H-1,2,4-triazol-1-yl)-2-(2,4-dichlorophenyl)-propan-2-ol (British patent application no. 2,078,719A, Example 2). The resulting mixture was heated under reflux for 2 hours. The oily residue, obtained after the evaporation of the solvent in vacuo, was partitioned between chloroform and aqueous sodium bicarbonate, the chloroform extract (40 ml) was successively washed with aqueous sodium bicarbonate (3 x 10 ml) and water, and dried ($MgSO_4$). Removal of the chloroform in vacuo,

followed by flash chromatography of the residue on silica gel (50 g) and elution with 95% ethyl acetate/5% diethylamine, yielded the title compound, which was crystallised from ethylacetate/hexane - 610 mg, m.p. 165°.

Analysis %:-

| | |
|---|---|
| Found: | C,43.5; H,3.1; N,23.8; |
| Calculated for $C_{13}H_{11}Cl_3N_6$: | C,43.6; H,3.1; N,23.5. |

Mass spectrometry confirmed the desired structure.

EXAMPLE 2

1,3-Bis(1H-1,2,4-triazol-1-yl)-2-bromo-2-(2,4-dichlorophenyl) propane

Method A:

A solution of 3.88 g of imidazole in 40 ml of dry acetonitrile was treated with 2 ml of thionyl bromide and then with 3.4 g of 1,3-bis(1H-1,2,4-triazol-1-yl)-2-(2,4-dichlorophenyl)-propan-2-ol. The resulting mixture was heated under reflux for 4 hours. The reaction mixture was then treated with a further 2 ml of thionyl bromide and heated under reflux for 20 hours. The residue obtained after evaporation of the solvent *in vacuo* was partitioned between methylene chloride and cold aqueous sodium bicarbonate, and the organic extract (100 ml) was successively washed with aqueous sodium bicarbonate (3 x 20 ml), water and dried ($MgSO_4$). Removal of methylene chloride followed by flash chromatography of the residue on silica (100 g) and

elution with 97% ethyl acetate/3% diethylamine yielded the title compound, which was crystallised from ethyl acetate/hexane, 430 mg, m.p. 162°.

Analysis %:-

| | |
|---|---|
| Found: | C,38.9; H,2.7; N,21.1. |
| $C_{13}H_{11}BrCl_2N_6$ requires: | C,38.8; H,2.7; N,20.9. |

Method B:

To an ice-cooled suspension of 1,3-bis(1H-1,2,4-triazol-1-yl)-2-(2,4-dichlorophenyl)-propan-2-ol (3.4 g) in dry acetonitrile (30 ml), thionyl bromide (1 ml) was added. After 2 minutes the ice bath was withdrawn and the reaction mixture was allowed to attain room temperature, thereby obtaining a clear solution from which, after 10 minutes, a solid crystallised out. This solid was collected by filtration and suspended in a mixture of methylene chloride (100 ml) and water (40 ml), cooled, and neutralised with solid sodium bicarbonate. The organic layer was separated, washed with water, and dried ($MgSO_4$). Removal of solvent yielded a solid which was crystallised from ethyl acetate/hexane to yield the title compound, 1.56 g, m.p. 162°, identical to the product of "Method A".

Analysis %:-

| | |
|---|---|
| Found: | C,38.7; H,2.7; N,21.2. |
| $C_{13}H_{11}BrCl_2N_6$ requires: | C,38.8; H,2.7; N,20.9. |

EXAMPLE 3

1,3-Bis(1H-1,2,4-triazol-1-yl)-2-bromo-2-(2,4-difluorophenyl)propane was prepared similarly to Example 2(B), using 1,3-bis(1H- 1,2,4-triazol-1-yl)-2-(2,4-difluorophenyl)propan-2-ol and thionyl bromide. It had an m.p. of 138-140°.

Analysis %:-

| | |
|---|---|
| Found: | C,42.4; H,3.1; N,22.6 |
| Calculated for $C_{13}H_{11}BrF_2N_6$: | C,42.3; H,3.0; N,22.8. |

EXAMPLE 4

1,3-Bis(1H-1,2,4-triazol-1-yl)-2-(2,4-dichlorophenyl)-2-fluoropropane

Diethylaminosulphurtrifluoride (2.112 g) was added to a suspension of 1,3-bis(1H-1,2,4-triazol-1-yl)-2-(2,4-dichlorophenyl)-propan-2-ol (3.4 g) in dry methylene chloride (20 ml) at 0°. After stirring at 0° for 1 hour, the resulting clear solution was treated with ice water (30 ml), allowed to come to room temperature, and stirred for a further 30 minutes. The mixture was then diluted with methylene chloride (50 ml), cooled again, and neutralised with solid sodium bicarbonate. The organic layer was separated, washed with water, and dried ($MgSO_4$). The residue obtained after the removal of the solvent was flash chromatographed on silica (100 g). Elution with 97% ethyl acetate/3% diethylamine gave the title compound, which was crystallised from ethyl acetate/hexane, yield 1.621 g, m.p. 150°.

Analysis %:-

Found: C,45.9; H,3.3; N,24.9

$C_{13}H_{11}Cl_2FN_6$ requires: C,45.8; H,3.2; N,24.6.

EXAMPLES 5 TO 8

The following compounds were prepared similarly to Example 1, starting from the appropriate 1,3-bis(1H-1,2,4-triazol-1-yl)-2-(substituted phenyl)propan-2-ol, imidazole and thionyl chloride.

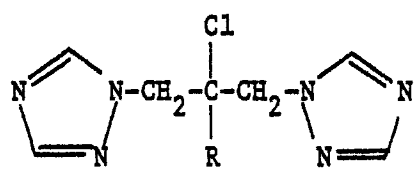

| Example No. | R | m.p. (°C) | Analysis % (Theoretical in brackets) C | H | N |
|---|---|---|---|---|---|
| 5 | 4-bromo-2,5-difluorophenyl | 220-225° | 38.7<br>(38.7 | 2.5<br>2.5 | 20.7<br>20.8) |
| 6 | 2,4-difluoro-phenyl | 159-161° | 48.3<br>(48.1 | 3.3<br>3.4 | 26.0<br>25.9) |
| 7 | 4-iodophenyl | 207-209° | 37.5<br>(37.7 | 2.9<br>2.9 | 20.2)<br>20.3) |
| 8 | 4-chlorophenyl | 169-171° | 48.2<br>(48.3 | 3.8<br>3.7 | 26.2<br>26.0) |

## EXAMPLE 9

1,3-Bis(1H-1,2,4-triazol-1-yl)-2-chloro-2-(5-chloropyrid-2-yl)propane

A solution of imidazole (0.43 g) in dry acetonitrile (4 ml) at 0° was treated with thionyl chloride (0.26 ml) and then with 1,3-bis-(1H-1,2,4-triazol-1-yl)-2-(5-chloropyrid-2-yl)-propan-2-ol (0.36 g). The resulting mixture was warmed to room temperature and stirred for 3 hours. The oily residue, obtained after evaporation of the solvent *in vacuo*, was partitioned between methylene chloride and aqueous sodium bicarbonate. The methylene chloride extract (20 ml) was dried ($MgSO_4$), filtered and evaporated. The residue was flash chromatographed on silica gel (45 g) eluting with 95% diethyl ether/5% methanol and the fractions containing the product were evaporated to yield the title compound as an oil (25 mg).

N.m.r. ($CDCl_3$,60MHz) $\delta$ = 5.02(4p,s); 7.6-7.75(2p,m); 7.9(1p,s); 8.21(1p,s); 8.4-8.5(1p,m).

The following Preparations illustrate the preparation of certain starting materials. All temperature are in °C.

## Preparation 1

Preparation of 1-[2-(2,4-difluorophenyl)-2-hydroxy-3-(1H-1,2,4-triazol-1-yl)-propyl]-1H-1,2,4-triazole

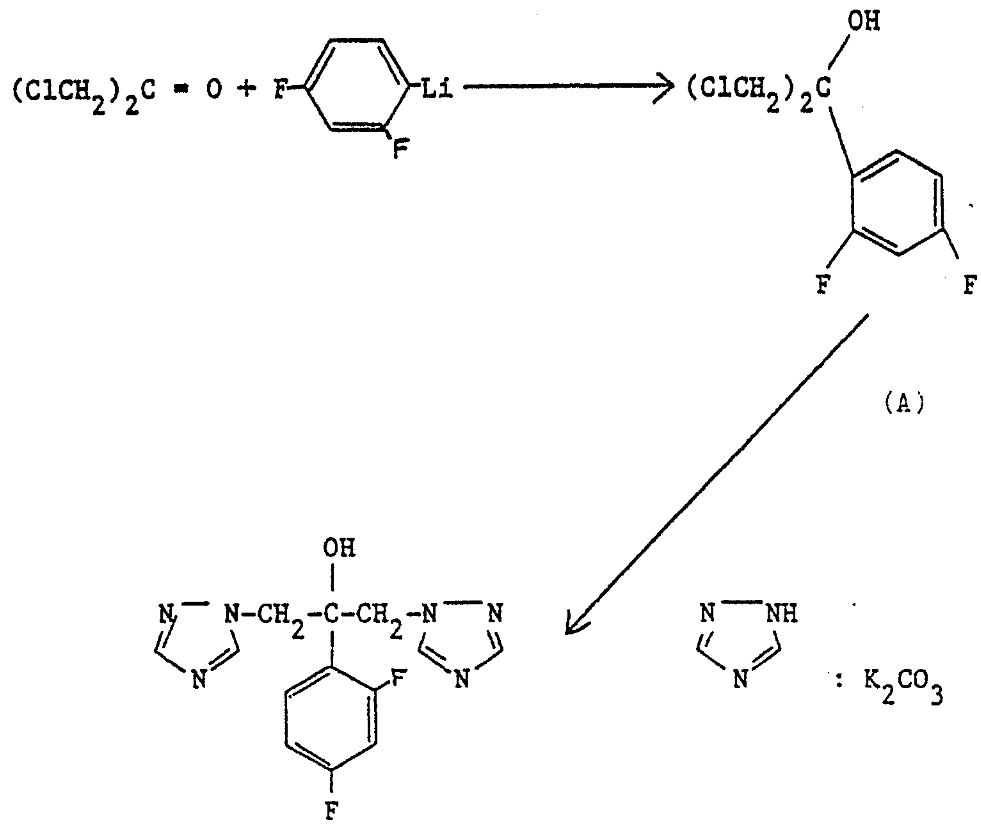


(i) A solution of 1-bromo-2,4-difluorobenzene (0.96 g, 5 mM) in diethyl ether (10 ml) was stirred at -78° and a solution of n-butyl lithium (1.55 M, 3.23 ml; 5 mM) in hexane was added over three minutes. After the addition was complete, the mixture was stirred for a further 10 minutes and then a solution of 1,3-dichloroacetone (0.63 g, 5 mM) in diethyl ether (10 ml) was added dropwise over three minutes. After stirring for a further 30 minutes at -78°, a solution of acetic acid (0.33 g) in diethyl ether (5 ml) was added at 0°, followed by water (10 ml). The organic layer was separated and the aqueous layer was washed once

with diethyl ether. The combined ether extracts were dried ($MgSO_4$) and evaporated to give a pale yellow oil which was dissolved in dimethyl formamide (20 ml). This DMF solution contained the intermediate (A).

(ii) 1,2,4-Triazole (1.72 g, 5 mM) and anhydrous potassium carbonate (2.07 g, 3 mM) were added to the solution prepared in (i) and the mixture was heated at 70° for 18 hours. The reaction mixture was cooled and poured into water (100 ml). The aqueous mixture was extracted twice with ethyl acetate. The combined organic extracts were dried ($MgSO_4$) and evaporated to give a gum. The gum was chromatographed on silica (270-400 Mesh), eluting with 3% methanol in methylene chloride to give the title compound, 1-[2-(2,4-difluorophenyl)-2-hydroxy-3-(1H-1,2,4-triazol-1-yl)-propyl]-1H-1,2,4-triazole as a white solid, 0.40 g (26% based on dichloroacetone), m.p. (after crystallisation from ethyl acetate/hexane), 138-140°.

Analysis %:-

| | |
|---|---|
| Found: | C,51.3; H,4.0; N,27.1. |
| Calculated for $C_{13}H_{12}F_2N_6O$: | C,51.0; H,3.9; N,27.4. |

'H,n.m.r., i.r. and mass spectral data were consistent with the required structure. The chromatographic procedure separated the desired product from the 1-[2-(2,4-difluorophenyl)-2-hydroxy-3-(4H-1,2,4-triazol-4-yl)-propyl]-1H-1,2,4-triazole impurity that was present in the reaction mixture.

## Preparations 2 and 3

The following compounds were prepared similarly to Preparation 1 starting from the appropriate 1,3-dichloro-2-(substituted phenyl)-propan-2-ol, potassium carbonate and 1,2,4-triazole:-

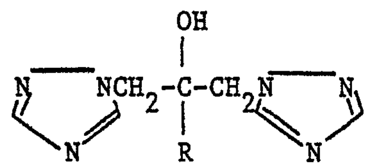

| Preparation No. | R | m.p. (°C) | Analysis % (Theoretical in brackets) C | H | N |
|---|---|---|---|---|---|
| 2 | 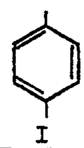 * | 164-6° | 38.8<br>(38.5 | 3.4<br>3.5 | 20.8<br>20.7) |
| 3 | 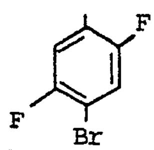 | 209-11° | 40.8<br>(40.5 | 2.9<br>2.9 | 22.3<br>21.8) |

The starting dichloro derivatives were prepared similarly to Preparation 1(i).

* Characterised as the hemihydrate.

## Preparation 4

1,3-Bis-(1H-1,2,4-triazol-1-yl)-2-(4-chlorophenyl)propan-2-ol monohydrate was prepared similarly to Example 2(D) of published U.K. patent application no. 2099818A, starting from

2-(4-chlorophenyl)-2-(1H-1,2,4-triazol-1-ylmethyl)oxirane (free base form), anhydrous potassium carbonate, and 1,2,4-triazole. It had an m.p. of 88-90°.

Analysis %:-

| | |
|---|---|
| Found: | C,48.3; H,4.7; N,26.2; |
| Calculated for $C_{13}H_{13}ClN_6O.H_2O$: | C,48.3; H,4.7; N,26.2. |

The oxirane starting material was prepared similarly to Example 2(C) of the said published U.K. application no. 2099818A from 4'-chloro-2-(1H-1,2,4-triazol-1-yl)acetophenone, trimethylsulphoxonium iodide and cetrimide in a mixture of toluene and sodium hydroxide. The ketone starting material was prepared similarly to U.K. patent specification no. 1,512,918 using acetone as the solvent at room temperature.

## Preparation 5

(a) Preparation of 2-(5-chloropyrid-2-yl)-1,3-dichloropropan-2-ol

A solution of n-butyl lithium in hexane (12.5 ml, 1.55 molar, 19.4 mmole) was added under nitrogen to a stirred solution of 2-bromo-5-chloropyridine (20 mmole) in dry diethyl ether (40 ml) at -78° over a period of 15 minutes. The mixture was stirred at -78° for a further 15 minutes after the addition was complete and a solution of 1,3-dichloroacetone (2.3 g, 20 mmole) in diethyl ether (25 ml) was then added over a period of 15 minutes. After a further 30 minutes at -78° a solution of acetic acid (2 ml) in diethyl ether (10 ml) was added followed by water (15 ml) and the

mixture allowed to warm to room temperature. The mixture was neutralised with aqueous sodium carbonate solution and the organic layer was separated and washed with water. The aqueous washes were extracted with diethyl ether and the combined organic layers were dried ($MgSO_4$) and evaporated to yield the title dichloropropanol derivative.

(b) Preparation of 1,3-bis(1H-1,2,4-triazol-1-yl)-2-(5-chloropyrid-2-yl)propan-2-ol

1,2,4-Triazole (3.3 g, 48 mmole), and anhydrous potassium carbonate (6.6 g, 48 mmole) were added to a solution of the said dichloropropanol derivative (20 mmole) in dry N,N-dimethylformamide (65 ml), and the mixture was heated on an oil bath at 80° for 2 hours with stirring. The mixture was cooled, the solvent evaporated under reduced pressure and the residue extracted with boiling ethyl acetate (3 x 50 ml). The combined extracts were evaporated to dryness and the residue was chromatographed on silica eluting with an appropriate solvent (a mixture of chloroform, methanol and concentrated ammonium hydroxide, 32:4:1). Fractions containing the product were combined and evaporated and the product recrystallised from an appropriate solvent to yield the title compound, m.p. 180-181°.

Analysis %:-

| | |
|---|---|
| Found: | C,47.1; H,3.9; N,32.2; |
| Calculated for $C_{12}H_{12}N_7OCl$: | C,47.1; H,4.0; N,32.1. |

## Preparation 6

### Preparation of 2-bromo-5-chloropyridine

2-Bromo-5-chloropyridine was prepared from 2-amino-5-chloropyridine according to the method of L. C. Craig, J. Amer. Chem. Soc., 1934, 56, 231, to furnish a white crystalline solid in 70% yield, m.p. 69-70°.

### Activity Data in mice (oral $PD_{50}$ values)

| Compound | $PD_{50}$ |
|---|---|
| Product of Example 1 | 0.1 |
| Product of Example 2 | 0.03 |
| Product of Example 3 | 0.3 |
| Product of Example 4 | 1.8 |
| Product of Example 5 | 1.6 |
| Product of Example 6 | 0.9 |
| Product of Example 7 | 0.2 |
| Product of Example 8 | 0.3 |
| Product of Example 9 | 0.2 |

CLAIMS (EXCEPT AT)

1. A compound of the formula:-

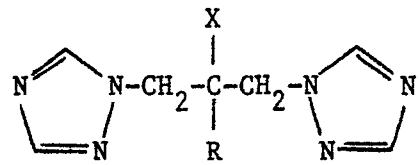

--- (I)

or a pharmaceutically or agriculturally acceptable salt thereof, wherein R is naphthyl, biphenylyl, or phenyl optionally substituted by 1 to 3 substituents each independently selected from F, Cl, Br, I, $CF_3$, $C_1$-$C_4$ alkyl and $C_1$-$C_4$ alkoxy; or R is a 5-chloropyrid-2-yl group;
and X is F, Cl or Br.

2. A compound as claimed in claim 1, wherein R is phenyl substitued by 4-chloro, 4-bromo, 4-fluoro, 4-iodo, 4-$CF_3$, 2-chloro, 2,4-dichloro, 2,4-difluoro, 2-chloro-4-fluoro, 2,5-difluoro, 2,4,6-trifluoro or 4-bromo-2,5-difluoro.

3. A compound as claimed in claim 1 wherein R is 2,4-dichlorophenyl, 2,4-difluorophenyl, 4-bromo-2,5-difluorophenyl, 4-iodophenyl, 4-chlorophenyl or 5-chloropyrid-2-yl.

4. 1,3-Bis(1H-1,2,4-triazol-1-yl)-2-bromo-2-(2,4-dichlorophenyl)propane.

5. A pharmaceutical composition comprising a compound of the formula (I) as claimed in any one of the preceding claims, or a pharmaceutically acceptable salt thereof, together with a pharmaceutically acceptable diluent or carrier.

6. A composition as claimed in claim 5, which is in the form of a tablet, capsule or injectable formulation.

7. A compound of the formula (I) as claimed in any one of claims 1 to 4, or a pharmaceutically acceptable salt thereof, for use in treating a fungal infection in a human being.

8. A process for preparing a compound of the formula (I) as claimed in claim 1, or a pharmaceutically acceptable salt thereof, which comprises reacting a compound of the formula:-

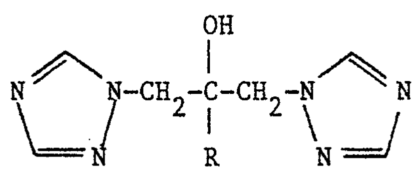

--- (II)

where R is as defined in claim 1,
with, as appropriate, thionyl chloride, thionyl bromide, or diethylaminosulphur trifluoride;
followed by, optionally, conversion of the product into a pharmaceutically acceptable salt.

9. A plant or seed antifungal composition comprising a compound of the formula (I) as claimed in any one of claims 1 to 4, or an agriculturally acceptable salt thereof, together with an agriculturally acceptable diluent or carrier.

10. A method of treating a plant or seed having a fungal infection, which comprises administering to said plant or seed an antifungally acceptable amount of a compound of the formula (I) as claimed in any one of claims 1 to 4, or an agriculturally acceptable salt thereof, or antifungally effective amount of a composition as claimed in claim 9.

PROCESS CLAIMS (AT)

1. A process for preparing a triazole of the formula:-

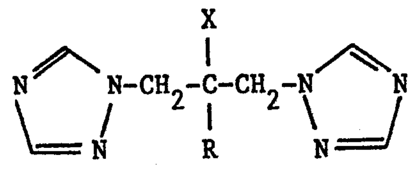

--- (I)

or a pharmaceutically or agriculturally acceptable salt thereof, wherein R is naphthyl, biphenylyl, or phenyl optionally substituted by 1 to 3 substituents each independently selected from F, Cl, Br, I, $CF_3$, $C_1$-$C_4$ alkyl and $C_1$-$C_4$ alkoxy; or R is a 5-chloropyrid-2-yl group;

and X is F, Cl or Br.

characterised by replacing the -OH group of a compound of the formula:-

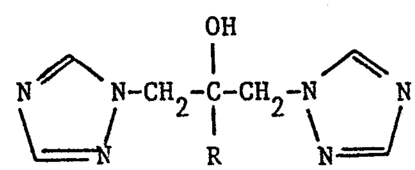

--- (II)

where R is as defined for formula (I),

with F, Cl or Br;

followed by, optionally, conversion of the product of the formula (I) into a pharmaceutically or agriculturally acceptable salt thereof.

2. A process according to claim 1, characterised by reacting the compound (II) with, as appropriate, diethylaminosulphurtrifluoride, thionyl chloride or thionyl bromide.

3. A process according to claim 2, characteirsed in that the reaction of the compound (II) with thionyl chloride or bromide is carried out in the presence of imidazole.

4. A process according to any one of the preceding claims, characterised in that a compound wherein R is phenyl substituted by 4-chloro, 4-bromo, 4-fluoro, 4-iodo, 4-$CF_3$, 2-chloro, 2,4-dichloro, 2,4-difluoro, 2-chloro-4-fluoro, 2,5-difluoro, 2,4,6-trifluoro or 4-bromo-2,5-difluoro, is prepared.

5. A process according to any one of claims 1 to 3, characterised in that a compound wherein R is 2,4-dichlorophenyl, 2,4-difluorophenyl, 4-bromo-2,5-difluorophenyl, 4-iodophenyl, 4-chlorophenyl or 5-chloropyrid-2-yl, is prepared.

6. A process according to claim 1, characterised in that 1,3-bis(1H-1,2,4-triazol-1-yl)-2-bromo-2-(2,4-dichlorophenyl)propane is prepared by reacting 1,3-bis(1H-1,2,4-triazol-1-yl)-2-(2,4-dichlorophenyl)propan-2-ol with thionyl bromide.

7. A process according to claim 6, characterised in that it is carried out in the presence of imidazole.

8. The use of a compound of the formula (I) as defined in claim 1, or an agriculturally acceptable salt thereof, as an agricultural fungicide.